# EUROPEAN PATENT APPLICATION

(11) **EP 4 119 078 A1**
(43) Date of publication of application: **18.01.2023**
(21) Application number: 21767457.1
(22) Date of filing: 09.03.2021
(51) Int. Cl.: A61B 17/86, A61B 17/80

(54) **SCREW ASSEMBLY FOR BONE FIXATION, AND BONE FIXATION DEVICE**

(30) Priority: 10.03.2020 CN 202010160874
(71) Applicant: Zoltrix Material International Limited, Hong Kong (HK)
(72) Inventor: QIN, Ling, Hong Kong (CN); CHAU, Wing Ho, Hong Kong (CN); LIU, Chi Hung Kevin, Hong Kong (CN); CHOW, Dick Ho Kiu, Hong Kong (CN)
(74) Representative: Hoffmann Eitle
(86) International application number: PCT/CN2021/079771
(87) International publication number: WO 2021/180076

(57) **Abstract**

A screw assembly (100) for bone (20) fixation and a bone fixation device (10). The screw assembly (100) for bone (20) fixation comprises a non-degradable nut (110) and a degradable and absorbable screw main body (120); the nut (110) is connected to one end of the screw main body (120) in a detachable manner; and the outer peripheral surface of the screw main body (120) has external threads. The bone fixation device (10) comprises a bone plate (200) and the screw assembly (100); several fixation through holes (210) penetrate through the bone plate (200) in the thickness direction of the bone plate; and after the screw main body (120) of the screw assembly (100) is fitted with one fixation through hole (210), the end of the screw main body (120) away from the nut (110) protrudes from the bone plate (200). The screw assembly (100) can be used for bone (20) fixation, and has stable mechanical properties and low cost. The screw assembly (100) is used for achieving bone (20) fixation by fitting with the bone plate (200), and can promote healing of bone fracture and accelerate remodeling or reconstruction of bone callus, avoiding delayed healing or non-healing of bone fracture.

## Description

### TECHNICAL FIELD

The present disclosure relates to the field of medical devices, in particular to a screw assembly for bone fixation and a bone fixation device.

### BACKGROUND

At present, most of the medical metals used clinically are non-degradable biocompatible materials such as stainless steel and titanium alloys. When the fracture fixation function of the implant in the human body is completed, in most cases, it needs to be surgically removed again. This procedure increases the financial and physical and mental burden of the patient.

Implants made of traditional medical metal materials have high mechanical properties and can achieve early rigid fixation, especially for load-bearing bone (e.g., femur, tibia) fractures. However, because the mechanical properties (e.g., density and elastic modulus) of the traditional medical metal do not match those of human bone, and the mechanical properties of the entire fixation system cannot change dynamically with the bone healing process, the "stress shielding effect" encountered in orthopedics occurs. This phenomenon leads to osteoporosis or bone disuse degeneration, which decrease the strength of the bone after healing.

The density and elastic modulus of pure magnesium (Mg) or magnesium alloys are similar and matched to those of human bone. This reduces or avoids the "stress shielding effect". These metals are also biodegradable and absorbable, and magnesium is an essential trace element for the human body. In addition, many scientific studies have found and proved that implants made of biodegradable and absorbable pure magnesium and magnesium alloys stimulate bone regeneration in the early stage of fracture healing and under the premise of a stable biomechanical environment, which has great potential in the treatment of osteoporotic fractures, osteonecrosis, and tendon-bone healing. However, the mechanical properties of the implants made of the biodegradable absorbent material (e.g., magnesium and magnesium alloys) are still insufficient. Since the elastic modulus of the magnesium is lower than that of traditional medical metal, deformation and fracture are more likely to occur. Magnesium is difficult to replace the application of the traditional metal implant for specific medical purposes (e.g., fractures of load-bearing bones).

### SUMMARY

Accordingly, it is necessary to provide a screw assembly for bone fixation and a bone fixation device with stable mechanical properties and low cost.

A screw assembly for bone fixation includes a non-degradable screw head and a degradable and absorbable screw body, wherein the screw head is detachably connected to an end of the screw body, and an outer peripheral surface of the screw body is provided with external threads.

In one of the embodiments, the screw assembly for bone fixation further includes a connecting element, wherein the screw head is detachably connected to the screw body through the connecting element.

In one of the embodiments, the connecting element is integrally connected to the screw head, the connecting element is provided with external threads, an end surface of the screw body mating with the screw head is provided with a matching groove with internal threads, and the connecting element is threadedly connected to the matching groove; or the connecting element is integrally connected to the screw body, the connecting element is provided with external threads, an end surface of the screw head mating with the screw body is provided with the matching groove with internal threads, and the connecting element is threadedly connected to the matching groove.

In one of the embodiments, when the connecting element is integrally connected to the screw head, the connecting element is non-degradable, when the connecting element is integrally connected to the screw body, the connecting element is degradable and absorbable.

In one of the embodiments, a sharp end of the screw body away from the screw head has a smooth curved surface structure.

In one of the embodiments, an end surface of the screw head away from the screw body is provided with a screw groove or a boss, the screw groove is a non-circular groove and the boss is a non-circular cylindrical structure.

In one of the embodiments, a head portion of an end of the screw head away from the screw body is cylindrical, spherical, conical, or spherical.

In one of the embodiments, an outer surface of the screw head is provided with a degradable or non-degradable film or coating, an outer surface of the screw body is provided with a degradable film or coating, when the connecting element is not degradable, an outer surface of the connecting element is provided with the degradable or non-degradable film or coating, when the connecting element is degradable and absorbable, an outer surface of the connecting element is provided with a degradable film or coating.

A bone fixation device includes a bone plate and the screw assembly for bone fixation, the bone plate is provided with a plurality of fixing through holes penetrating through the bone plate along a thickness direction thereof, after the screw body of the screw assembly cooperates with the fixing through hole, an end of the screw body away from the screw head protrudes from the bone plate.

In one of the embodiments, the fixing through hole is provided with the internal thread, an outer peripheral surface of the end of the screw head adjacent to the screw body is provided with the external thread, the external thread of the screw head cooperates with the internal thread of the fixing through hole.

In one of the embodiments, the fixing through hole is a countersunk hole, and the screw head is cooperated with the fixing through hole, after the screw head cooperates with the fixing through hole, the screw head is flush with the bone plate or the screw head is located in the fixing through hole.

In one of the embodiments, the bone fixation device further includes a non-degradable screw configured to cooperate with the fixing through hole.

In one of the embodiments, the non-degradable screw is composed of a non-degradable biocompatible material.

In one of the embodiments, a sharp end of the non-degradable screw has a smooth curved surface structure.

In one of the embodiments, the surface of the bone plate for cooperating with the bone is provided with a plurality of recessed abdicating grooves, and the fixing through holes are misaligned with the abdicating grooves.

In one of the embodiments, an outer surface of the bone plate and the outer surface of the screw head is provided with the degradable or non-degradable film or coating, the outer surface of the screw body is provided with a degradable film or coating, when the connecting element is not degradable, the outer surface of the connecting element is provided with the degradable or non-degradable film or coating, when the connecting element is degradable and absorbable, the outer surface of the connecting element is provided with the degradable film or coating.

The screw assembly of the present disclosure can be used for bone fixation with stable mechanical properties and low cost. The screw assembly is configured to cooperate with a bone plate to fix the bone and help the healing of the fracture bone. The degradation products released after the biodegradable and resorbable screw body is degraded will stimulate the surrounding bone and tissue and promote the formation of new bone. With the degradation of the screw body, the rigidity of a fracture fixation structure will gradually decrease, which can dynamically fix a fracture, or avoid or reduce the "stress shielding effect" caused by the traditional rigid "screw-plate" system. Therefore, the load on the bone near the fracture will gradually increase, which can stimulate fracture healing and accelerate bone regeneration, at the same time avoid delayed union or nonunion of the fracture, and achieve better bone healing and remodeling.

The screw head in the screw assembly for bone fixation of the present disclosure is made of non-degradable biocompatible materials with strong mechanical properties such as stainless steel, titanium alloy, etc., which can ensure the strength of the entire screw assembly and enable the screw head to withstand large torsion without wear and slippage to achieve the desired holding stability. The screw body is a magnesium or magnesium alloy screw, which degrades near the fracture and releases magnesium ions and raises the local pH value to become alkaline, which is beneficial to the growth of new bone near the fracture and fracture healing. As the screw body degrades, new bone tissue forms at the position of the degraded original screw body and fills the gap at the position, which can reduce the risk of re-fracture during and after the implant removal operation in the future.

The screw head of the screw assembly for bone fixation of the present disclosure uses a non-circular groove such as a quincunx-shaped groove, a star-shaped groove, a hexagonal groove, etc., or a convex column arranged in a non-circular cylindrical structure, so that the screw head can withstand large torque without abrasion and sliding.

The bone fixation device of the present disclosure has stable mechanical properties, which can realize the bone fixation, and help the fracture to be quickly repaired and healed.

In the bone fixation device of the present disclosure, the external thread of the screw head is cooperated with the internal thread of the fixing through hole of the bone plate, so to achieve a mutual locking of the screw head and the bone plate to obtain a more rigid and stable structure.

The surface of the bone plate of the bone fixation device of the present disclosure for cooperating with the bone is provided with the plurality of recessed abdicating grooves, and the fixing through holes are misaligned with the abdicating grooves, so that the bone plate does not fit on the surface of the bone and does not need to be pressed against the bone, which can reduce the damage of the bone plate to the periosteal blood supply and hinder the formation of callus.

The fixing through hole of the bone fixation device of the present disclosure is a countersunk hole, the screw head is cooperated with the fixing through hole, and after the screw head cooperates with the fixing through hole, the screw head is flush with the bone plate or the screw head is located in the fixing through hole. Therefore, the screw head will not be protruded and can not stimulate the soft tissue around the bone, which avoid discomfort and pain of the operator after the implantation operation.

The bone fixation device of the present disclosure further uses non-degradable screws. The non-degradable screws and screw assembly are used in combination to fix the bone plate to the bone to achieve the desired stability of the fracture. The screw assembly is used near the fracture to achieve later degradation and promote fracture healing. The non-degradable screw is used far from the fracture to achieve the mechanical strength and the stability required by the fracture fixation structure, and continue to fix the bone plate on the bone after the screw body is degraded, so as to prevent displacement of the bone plate.

The screw head of the bone fixation device of the present disclosure has an external thread on the outer peripheral surface of one end of the screw head adjacent to the screw body, and the external thread of the screw head cooperates with the internal thread in the fixing through hole, when the screw body is completely degraded, the non-degradable screw head will continue to be locked on the bone plate without loosening.

### BRIEF DESCRIPTION OF THE DRAWINGS

FIG. 1 is a cross-sectional view of a screw assembly for bone fixation according to a first embodiment of the present disclosure.
FIG. 2 is a top view of the screw assembly for bone fixation shown in FIG. 1.
FIG. 3 is a cross-sectional view of the screw assembly for bone fixation according to the first embodiment of the present disclosure.
FIG. 4 is a cross-sectional view of a bone fixation device according to a second embodiment of the present disclosure.
FIG. 5 is a schematic cross-sectional view of the bone fixation device according to the second embodiment of the present disclosure.

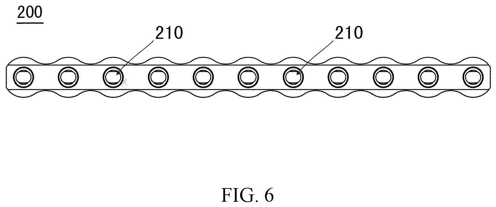

FIG. 6 is a top view of a bone plate according to the second embodiment of the present disclosure.

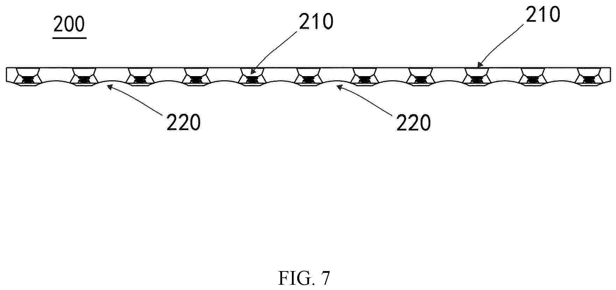

FIG. 7 is a cross-sectional view of the bone plate according to the second embodiment of the present disclosure.
FIG. 8 is a schematic diagram of the bone fixation device fixed to the bone shown in FIG. 5.
FIG. 9 is a schematic diagram of the bone fixation device and a screw body shown in FIG. 8 after degradation.

10: bone fixation device; 100: screw assembly; 110: screw head; 111: screw groove; 120: screw body; 130: connecting element; 200: bone plate; 210: fixation through hole; 220: abdicating groove; 300: non-degradable screw; 20: bone.

### DETAILED DESCRIPTION OF THE EMBODIMENTS

The present disclosure will now be described in detail with reference to the accompanying drawings and embodiments in order to understand the present disclosure more clear. Preferred embodiments of the present disclosure are shown in the accompanying drawings. However, preferred embodiments of the present disclosure is shown in the drawings. However, the present disclosure may be implemented in many different forms and is not limited to the embodiments described herein. On the contrary, the embodiments are provided for a more thorough and comprehensive understanding of the present disclosure.

It should be noted that when an element is referred to as being "fixed on" another element, it can be directly on the other element or an intervening element may also be present. When an element is referred to as being "connected" to another element, it can be directly connected to the other element or intervening elements may also be present. When one element is considered to be "mounted on" to another element, it can be directly connected to the other element or intervening elements may also be present. When one element is considered to be "disposed on" to another element, it can be directly connected to the other element or intervening elements may also be present.

Unless otherwise defined, all technical and scientific terms used herein have the same meaning as commonly understood by one of ordinary skill in the art to which this disclosure belongs. The terms used herein in the description of the present disclosure are for the purpose of describing specific embodiments only, and are not intended to limit the present disclosure. As used herein, the term "and/or" includes any and all combinations of one or more of the associated listed items.

### First embodiment

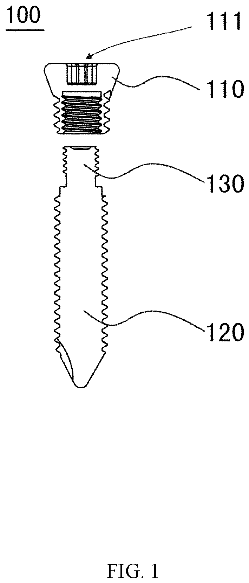

Referring to FIG. 1, a screw assembly 100 for bone fixation is provided according to the present embodiment. The screw assembly 100 for bone fixation includes a non-degradable screw head 110 and a degradable absorbable screw body 120. The screw head 110 is detachably connected to an end of the screw body 120, and an outer peripheral surface of the screw body 120 is provided with external threads. The screw head 110 may be made of a non-degradable material having strong mechanical properties, such as stainless steel and titanium alloy.

The screw assembly 100 for bone fixation further includes a connecting element 130. The screw head 110 is detachably connected to the screw body 120 through the connecting element 130.

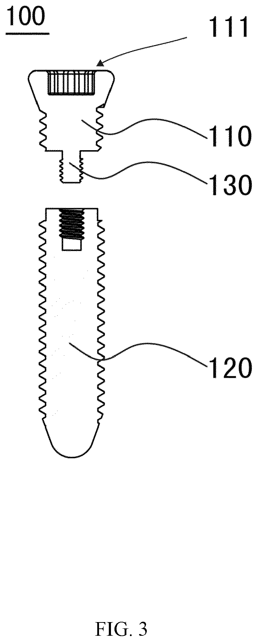

As shown in FIG. 3, the connecting element 130 is integrally connected to the screw head 110. The connecting element 130 is provided with external threads, an end surface of the screw body 120 mating with the screw head 110 is provided with a matching groove with internal threads, and the connecting element 130 is threadedly connected to the matching groove. Alternatively, as shown in FIG. 1, the connecting element 130 is integrally connected to the screw body 120. The connecting element 130 is provided with external threads, an end surface of the screw head 110 mating with the screw body 120 is provided with the matching groove with internal threads, and the connecting element 130 is threadedly connected to the matching groove.

The connecting element 130 may be made of a degradable absorbent material or a non-degradable material.

When the connecting element 130 is integrally connected to the screw head 110, the connecting element 130 is non-degradable. When the connecting element 130 is integrally connected to the screw body 120, the connecting element 130 is degradable and absorbable. The connecting element 130 is made of degradable absorbing material, for example, the connecting element 130 is a magnesium column.

The screw body 120 is made of a degradable absorbent material. For example, the screw body 120 is a magnesium or magnesium alloy screw.

Further, a sharp end of the screw body 120 away from the screw head 110 can be in any shape. Preferably, please referring to FIG. 1 and FIG. 3, the sharp end of the screw body 120 away from the screw head 110 has a smooth curved surface structure. The smooth curved surface structure of the screw body 120 away from the sharp end of the screw head 110 can prevent the screw body 120 from irritating soft tissue around the bone after the screw body 120 is implanted.

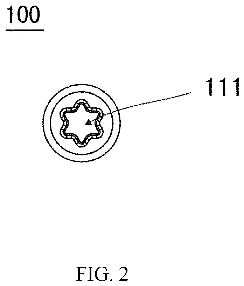

Further, as shown in FIG. 2, an end surface of the screw head 110 away from the screw body 120 is provided with a screw groove 111 or a boss (not shown). The screw groove 111 is a non-circular groove, and the boss is a non-circular cylindrical structure. The screw head 110 of the screw assembly 100 for bone fixation of the present disclosure is provided with a non-circular groove, such as a quincunx groove, a star groove, a hexagonal groove, etc., or a non-circular columnar structure convex column, so that the screw head 110 can withstand a large torque without wear and slippage.

Further, a head portion of an end of the screw head 110 away from the screw body 120 is cylindrical, spherical, conical, or spherical.

Preferably, an outer surface of the screw head 110 is provided with a degradable or non-degradable film or coating, an outer surface of the screw body 120 is provided with a degradable film or coating. When the connecting element 130 is not degradable, an outer surface of the connecting element 130 is provided with the degradable or non-degradable film or coating. When the connecting element 130 is degradable and absorbable, the outer surface of the connecting element 130 is provided with the degradable film or coating. For example, when the screw body 120 and the connecting element 130 are made of magnesium or magnesium alloy materials, the outer surface of the screw body 120 and the outer surface of the connecting element 130 form a degradable and biocompatible film, which can regulate the degradation rate of magnesium or magnesium alloy, improve the mechanical properties of parts made of magnesium or magnesium alloy, and can avoid electrochemical reactions caused by direct contact of two materials with different electrode potentials, and avoid accelerated corrosion degradation of the parts made of magnesium or magnesium alloy. The film/coating can be oxide films (e.g., MgO), ceramic materials (e.g., hydroxyapatite (HA)), polymers (e.g., polylactic acid (PLA)).

The screw assembly 100 of the present disclosure can be used for bone fixation with stable mechanical properties and low cost. The screw assembly 100 is configured to cooperate with a bone plate 200 to fix a bone 20 and help the healing of the fracture bone 20. After the biodegradable and absorbable screw body 120 is partially degraded, the released degradation products will stimulate the surrounding bone 20 and tissue and promote the formation of new bone. With the degradation of the screw body 120, the rigidity of a fracture fixation structure will gradually decrease, which can dynamically fix a fracture, and can avoid or reduce the "stress shielding effect" caused by the traditional rigid "screw-plate" system. Therefore, the load on the bone 20 near the fracture will gradually increase, so as to realize the gradual transmission of the force at the fracture healing, promote the fracture healing and accelerate the remodeling or reconstruction of the callus, avoid the delayed healing or nonunion of the fracture, and achieve a better repair effect of the bone 20.

The screw head 110 in the screw assembly 100 for bone fixation of the present disclosure is made of non-degradable biocompatible materials with strong mechanical properties such as stainless steel, titanium alloy, etc., which can ensure the strength of the entire screw assembly 100 and enable the screw head 110 to withstand large torsion without wear and slippage to achieve the desired holding stability. The screw body 120 is a magnesium or magnesium alloy screw, which degrades near the fracture and releases magnesium ions and raises the local pH value to become alkaline, which is beneficial to the growth of new bone near the fracture and fracture healing. As the screw body 120 degrades, new bone tissue forms at the position of the degraded original screw body 120 and fills the gap at the position, which can reduce the risk of re-fracture during and after the implant removal operation in the future.

### Second embodiment

A bone fixation device 10 is provided according to the present embodiment.

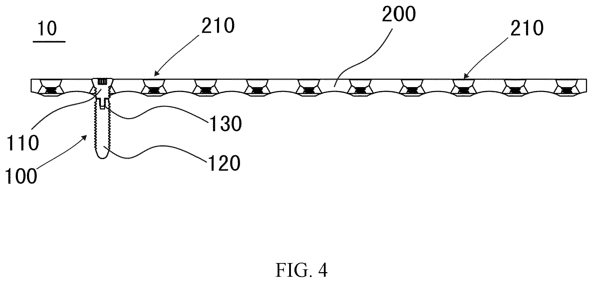

The bone fixation device 10 includes the bone plate 200 and the screw assembly 100 for bone fixation described in the first embodiment. Referring to FIG. 4, the bone plate 200 is provided with a plurality of fixing through holes 210 penetrating through the bone plate 200 along a thickness direction thereof, and the screw body 120 is configured to cooperate with the fixing through holes 210. After the screw body 120 of the screw assembly 100 extends through and cooperates with the fixing through hole 210, the end of the screw body 120 away from the screw head 110 protrudes from the bone plate 200, so that the screw body 120 can be inserted into the bone 20 after extending through the bone plate 200. Preferably, the length of the screw body 120 is greater than the thickness of the bone plate 200.

Preferably, the inner diameter of the fixing through hole 210 is less than the maximum outer diameter of the head portion of the screw head 110.

The bone fixation device 10 of the present disclosure has stable mechanical properties, which can realize the fixation of the bone 20, and help the fracture to be quickly repaired and healed.

Further, please also refer to FIG. 3, an outer peripheral surface of the end of the screw head 110 adjacent to the screw body 120 is provided with the external threads, and the fixing through hole 210 is provided with the internal threads. Referring to FIG. 4, a cooperation of the external threads of the screw head 110 and the internal threads of the fixing through hole 210 can achieve a mutual locking of the screw head 110 and the bone plate 200, so as to obtain a more rigid and stable structure. In addition, when the screw body 120 is completely degraded, the non-degradable screw head 110 will continue to be locked on the bone plate 200 and will not be loosened.

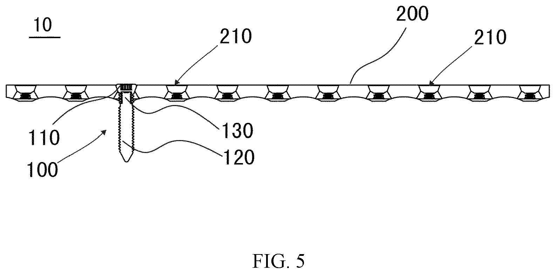

Further, referring to FIG. 4 and FIG. 5, the fixing through hole 210 is a countersunk hole, and the screw head 110 is cooperated with the fixing through hole 210. After the screw head 110 cooperates with the fixing through hole 210, the screw head 110 is coplanar with the bone plate 200 or the screw head 110 is located in the fixing through hole 210, that is, the screw head 110 is lower than the surface of the bone plate 200. In this way, the screw head 110 that is not protruded can prevent the screw head 110 from stimulating the soft tissue around the bone and avoid discomfort and pain of the operator after the implantation operation.

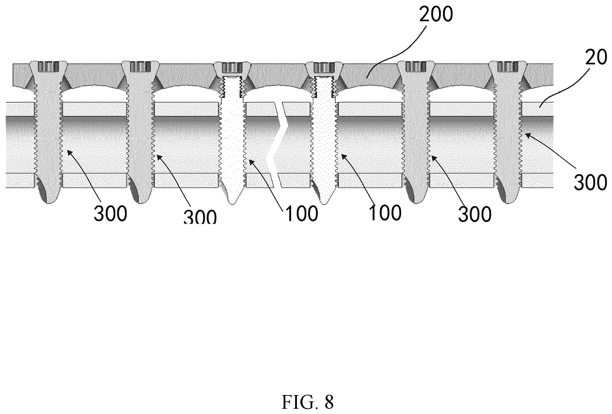

Further, the bone fixation device 10 further includes a non-degradable screw 300. The non-degradable screw 300 is configured to cooperate with the fixing through hole 210. In the bone fixation device 10 of the present disclosure, the non-degradable screw 300 and the screw assembly 100 are used in combination to fix the bone plate 200 to the bone to achieve desired fracture fixation stability. Referring to FIG. 8, the screw assembly 100 of the first embodiment is used near the fracture to achieve later degradation and promote fracture healing. The non-degradable screw 300 is used far away from the fracture to obtain the mechanical strength and the stability required by the fracture fixation structure, and continue to fix the bone plate 200 on the bone after the screw body 120 is degraded, so as to prevent displacement of the bone plate 200.

Further, the non-degradable screw 300 is made of non-degradable metal. For example, the non-degradable screw 300 is a titanium screw. It should be understood that the non-degradable screw 300 may also be composed of a non-degradable biocompatible metal such as titanium alloy and stainless steel, etc.

Further, a sharp end of the non-degradable screw 300 can be in any shape. Preferably, in an embodiment, the sharp end of the non-degradable screw 300 has a smooth curved surface structure. The smooth curved surface structure of the sharp end of the non-degradable screw 300 can prevent prevents the non-degradable screw 300 from irritating the soft tissue surrounding the bone after the non-degradable screw 300 is implanted.

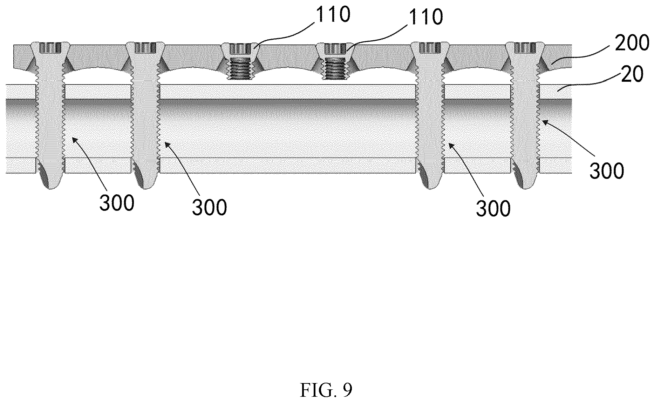

Further, the surface of the bone plate 200 for cooperating with the bone 20 is provided with a plurality of recessed abdicating grooves 220, and the fixing through holes 210 are misaligned with the abdicating grooves 220. Also referring to FIG.8 and FIG.9, according to the present disclosure, the surface of the bone plate 200 is provided with the plurality of recessed abdicating grooves 220, and the fixing through holes 210 are misaligned with the abdicating grooves 220, so that the bone plate 200 is not attached to the surface of the bone and does not need to press against the bone, which can reduce the damage of the bone plate 200 to the periosteal blood supply and hinder the formation of callus.

Preferably, the outer surface of the bone plate 200 and the outer surface of the screw head 110 is provided with the degradable or non-degradable film or coating, the outer surface of the screw body 120 is provided with the degradable film or coating. When the connecting element 130 is not degradable, the outer surface of the connecting element 130 is provided with the degradable or non-degradable film or coating. When the connecting element 130 is degradable and absorbable, the outer surface of the connecting element 130 is provided with the degradable film or coating. For example, when the screw body 120 and the connecting element 130 are made of magnesium or magnesium alloy materials, the outer surface of the screw body 120 and the outer surface of the connecting element 130 form a degradable and biocompatible film, which can regulate the degradation rate of magnesium or magnesium alloy, improve the mechanical properties of parts made of magnesium or magnesium alloy, and can avoid electrochemical reactions caused by direct contact of two materials with different electrode potentials, and avoid accelerated corrosion degradation of the parts made of magnesium or magnesium alloy. The film/coating can be oxide films (e.g., MgO), ceramic materials (e.g., hydroxyapatite (HA)), polymers (e.g., polylactic acid (PLA)).

The technical features of the above embodiments can be combined arbitrarily. For the sake of brevity, all possible combinations of the technical features in the above embodiments are not described. However, as long as there is no contradiction in the combination of these technical features, it should be considered as the scope of this description.

The foregoing descriptions are merely specific embodiments of the present disclosure, but are not intended to limit the protection scope of the present disclosure. It should be pointed out that any variation or replacement readily figured out by a person skilled in the art within the technical scope disclosed in the present disclosure shall all fall within the protection scope of the present disclosure. Therefore, the protection scope of the present disclosure shall be subject to the protection scope of the appended claims.

## Claims

1. A screw assembly for bone fixation, comprising a non-degradable screw head and a degradable and absorbable screw body, wherein the screw head is detachably connected to an end of the screw body, and an outer peripheral surface of the screw body is provided with external threads.

2. The screw assembly for bone fixation according to claim 1, further comprising a connecting element, wherein the screw head is detachably connected to the screw body through the connecting element.

3. The screw assembly for bone fixation according to claim 2, wherein the connecting element is integrally connected to the screw head, the connecting element is provided with external threads, an end surface of the screw body mating with the screw head is provided with a matching groove with internal threads, and the connecting element is threadedly connected to the matching groove;
or the connecting element is integrally connected to the screw body, the connecting element is provided with external threads, an end surface of the screw head mating with the screw body is provided with the matching groove with internal threads, and the connecting element is threadedly connected to the matching groove.

4. The screw assembly for bone fixation according to claim 2, wherein when the connecting element is integrally connected to the screw head, the connecting element is non-degradable, when the connecting element is integrally connected to the screw body, the connecting element is degradable and absorbable.

5. The screw assembly for bone fixation according to any one of claims 1 to 4, wherein a sharp end of the screw body away from the screw head has a smooth curved surface structure.

6. The screw assembly for bone fixation according to any one of claims 1 to 4, wherein an end surface of the screw head away from the screw body is provided with a screw groove or a boss, the screw groove is a non-circular groove and the boss is a non-circular cylindrical structure.

7. The screw assembly for bone fixation according to any one of claims 1 to 4, wherein a head portion of an end of the screw head away from the screw body is cylindrical, spherical, conical, or spherical.

8. The screw assembly for bone fixation according to any one of claims 2 to 4, wherein an outer surface of the screw head is provided with a degradable or non-degradable film or coating, an outer surface of the screw body is provided with a degradable film or coating, when the connecting element is not degradable, an outer surface of the connecting element is provided with the degradable or non-degradable film or coating, when the connecting element is degradable and absorbable, an outer surface of the connecting element is provided with a degradable film or coating.

9. A bone fixation device comprising a bone plate and the screw assembly for bone fixation according to any one of claims 1 to 8, wherein the bone plate is provided with a plurality of fixing through holes penetrating through the bone plate along a thickness direction thereof, after the screw body of the screw assembly cooperates with the fixing through hole, an end of the screw body away from the screw head protrudes from the bone plate.

10. The bone fixation device according to claim 9, wherein the fixing through hole is provided with the internal thread, an outer peripheral surface of the end of the screw head adjacent to the screw body is provided with the external thread, the external thread of the screw head cooperates with the internal thread of the fixing through hole.

11. The bone fixation device according to claim 9, wherein the fixing through hole is a countersunk hole, and the screw head is cooperated with the fixing through hole, after the screw head cooperates with the fixing through hole, the screw head is flush with the bone plate or the screw head is located in the fixing through hole.

12. The bone fixation device according to any one of claims 9 to 11, further comprising a non-degradable screw configured to cooperate with the fixing through hole.

13. The bone fixation device according to claim 12, wherein the non-degradable screw is composed of a non-degradable biocompatible material.

14. The bone fixation device according to claim 12, wherein a sharp end of the non-degradable screw has a smooth curved surface structure.

15. The bone fixation device according to any one of claims 9 to 11, wherein the surface of the bone plate for cooperating with the bone is provided with a plurality of recessed abdicating grooves, and the fixing through holes are misaligned with the abdicating grooves.

16. The bone fixation device according to any one of claims 9 to 11, wherein an outer surface of the bone plate and the outer surface of the screw head is provided with the degradable or non-degradable film or coating, the outer surface of the screw body is provided with a degradable film or coating, when the connecting element is not degradable, the outer surface of the connecting element is provided with the degradable or non-degradable film or coating, when the connecting element is degradable and absorbable, the outer surface of the connecting element is provided with the degradable film or coating.
